# EUROPÄISCHE PATENTSCHRIFT

(11) **EP 1 264 167 B1**
(45) Veröffentlichungstag und Bekanntmachung des Hinweises auf die Patenterteilung: **27.04.2005**
(21) Anmeldenummer: 01913647.2
(22) Anmeldetag: 15.02.2001
(51) Int. Cl.: G01N 21/64, G01N 27/26, B01J 19/00

(54) **VORRICHTUNG UND VERFAHREN ZUM NACHWEIS ORGANISCHER MOLEKÜLE IN EINER PROBENSUBSTANZ**
DEVICE AND METHOD FOR DETECTING ORGANIC MOLECULES IN A TEST SUBSTANCE
DISPOSITIF ET PROCEDE POUR LA DETECTION DE MOLECULES ORGANIQUES DANS UNE SUBSTANCE D'ESSAI

(30) Priorität: 17.03.2000 DE 10013254
(43) Veröffentlichungstag der Anmeldung: 11.12.2002
(73) Patentinhaber: Lossau, Harald, Dr., 80469 München (DE)
(72) Erfinder: HARTWICH, Gerhard, 80639 München (DE); LOSSAU, Harald, 81667 München (DE)
(74) Vertreter: Kritzenberger & Zeuner
(86) Internationale Anmeldenummer: PCT/DE2001/000571
(87) Internationale Veröffentlichungsnummer: WO 2001/069210

(56) Entgegenhaltungen:
- WO-A-00/13784
- DE-A- 19 940 751
- DE-A- 19 940 752
- US-A- 5 936 730
- US-A- 5 985 568

## Beschreibung

### Technisches Gebiet

Die vorliegende Erfindung betrifft eine Vorrichtung und ein Verfahren zum Nachweis von organischen Molekülen, insbesondere Biomolekülen und Polymeren, in einer Probensubstanz.

### Stand der Technik

Bio-Sensor-Chips werden insbesondere in den Ausführungsformen DNA-Chip und Protein-Chip in der biotechnologischen und genetischen Forschung eingesetzt. Ihnen wird ein großes Anwendungspotential in der medizinischen Diagnostik, pharmakologischen und toxikologischen Testverfahren sowie im Agrarsektor zugesprochen.

Ein Bio-Sensor-Chip enthält typischerweise ein zweidimensionales Array von Bereichen mit organischen Molekülen (Sonden), die auf einer Oberfläche fixiert sind und die mit chemischen Substanzen aus einer Probensubstanz (Targets) spezifisch reagieren können. Ziel ist dabei der parallele Nachweis vieler Wechselwirkungen zwischen Sonden und Targets. Lichtempfindliche Bio-Sensor-Chips zeigen bei Beleuchtung eine physikalische Reaktion, die spezifisch von der Wechselwirkung zwischen Sonden und Targets abhängt.

Die Detektion dieser Wechselwirkungen geschieht beispielsweise durch optische, autoradiographische, massenspektroskopische oder elektrische Verfahren. Dabei ist eine räumliche Adressierung der verschiedenen Sonden auf dem Array erforderlich. Innerhalb der Gruppe der lichtempfindlichen Bio-Sensor-Chips sind bisher insbesondere optische (Lumineszenz-) und elektrische Detektionsverfahren bekannt. Die Adressierung ist bei diesen Chips durch eine räumlich begrenzte optische Anregung erreichbar.

Für die optische Anregung werden bisher Laser Scanner oder konfokale Mikroskope eingesetzt. Kritische Parameter dabei sind Uniformität und Reproduzierbarkeit über den gesamten räumlichen Auslesebereich des Chips. Aufgrund dieser Anforderungen wird oft der ursprüngliche konfokale Aufbau von Minsky (US 3,013,467) als Grundlage für Auslesegeräte, wie beispielsweise in US 5,631,734 und US 5,578,832 beschrieben, verwendet. Er besteht aus einem xyz-Verschiebetisch, der unter einem konfokalen Mikroskop verschoben werden kann. In JP11094747 wird ein rotierender Tisch anstatt eines Verschiebetischs verwendet. Bei diesen Verfahren muss der Bio-Sensor-Chip den auftretenden Beschleunigungen des Tisches standhalten. Dies bestimmt letztendlich die Zeitdauer, in der der Chip ausgelesen werden kann. Beispielsweise wird für einen Scan-Bereich von 22 x 60 mm mit einer Auflösung von 10 µm für den Auslesevorgang etwa eine halbe Stunde benötigt.

Andere optische Auslesesysteme, wie beispielsweise in WO09947964A1 beschrieben, basieren auf beweglichen optischen Bauteilen, wodurch der Aufbau aufwendig und/oder störanfällig wird.

Ein spezielles Auslesesystem für lichtempfindliche Bio-Sensor-Chips mit elektrischem Ausleseverfahren ist bisher nicht bekannt.

Nachteilig an allen bekannten Abrastsystemen ist, dass sie mechanisch anfällig, relativ groß und teuer sind.

Die Druckschrift WO 00/13784 offenbart einen Analysator für Biomoleküle mit einem Array getrennt ansteuerbarer Lichtquellen und einer auf dem Lichtquellenarray angeordneten photoleitenden Schicht, deren dem Lichtquellenarray zugewandte Oberfläche mit einer leitfähigen Schicht versehen ist. Das Lichtquellenarray definiert auf der gegenüberliegenden Oberfläche der photoleitenden Schicht eine Mehrzahl von Teststellen. Jeder Teststelle eines Teilarrays von Teststellen ist zumindest eine, im Wesentlichen nur sie beleuchtende Beleuchtungsquelle zugeordnet. Eine Lösung mit Biomolekülen wird in elektrischen Kontakt mit den Teststellen gebracht und ein elektrisches Potential zwischen der Lösung und der elektrisch leitenden Schicht angelegt. Durch lokale Beleuchtung eines Teilbereichs der photoleitenden Schicht mit den Lichtquellen wird jeweils ein elektrischer Stromkreis zwischen der Lösung und der elektrisch leitenden Schicht geschlossen Photoleitende und leitfähige Schicht dienen als Detektionssystem ohne räumliche Auflösung.

### Darstellung der Erfindung

Aufgabe der vorliegenden Erfindung ist es deshalb, eine Vorrichtung und ein Verfahren zum Nachweis von organischen Molekülen, insbesondere Biomolekülen und Polymeren, in einer Probensubstanz oder deren Wirkung auf ein Untersuchungssystem zu schaffen, die die Nachteile des Standes der Technik nicht aufweisen.

Diese Aufgabe wird erfindungsgemäß durch das Verfahren gemäß unabhängigem Patentanspruch 1 sowie durch die Vorrichtung gemäß unabhängigem Patentanspruch 12 gelöst. Bevorzugte Ausführungsformen sind in den abhängigen Ansprüchen angegeben.

Die erfindungsgemäße Vorrichtung umfasst ein Untersuchungssystem aus einem Array von Teststellen, denen die Probensubstanz zuführbar ist, ein Beleuchtungssystem und ein Detektionssystem.

### Untersuchungssystem

In der vorliegenden Beschreibung wird der Begriff Bio-Sensor-Chip für ein Untersuchungssystem aus einem zweidimensionalen Array von Teststellen verwendet, wobei jede Teststelle spezifische Sondenmoleküle aufweist. Dabei sind die Sondenmoleküle auf einer Oberfläche fixiert und können mit chemischen Substanzen aus einer Probensubstanz (Targets) spezifisch reagieren. Ziel ist dabei der parallele Nachweis vieler Wechselwirkungen zwischen Sonden und Targets.

Der Begriff Sondenmoleküle umfasst insbesondere Biomoleküle, Polymere und deren Komplexe mit anderen chemischen Substanzen, insbesondere Biomolekülen, Polymeren, Farbstoffen, Metallen und redoxaktive Substanzen. Sondenmoleküle umfassen bevorzugt DNA-, RNA- oder PNA-Fragmente (DNA - Desoxyribonukleinsäure, RNA - Ribonukleinsäure, PNA - Peptidnukleinsäure: Synthetische DNA oder RNA, bei der die Zucker-Phosphat-Einheit durch eine Aminosäure ersetzt ist). Der entsprechende Bio-Sensor-Chip wird in diesen Fällen mit DNA-Chip bezeichnet. Ebenfalls bevorzugte Sondenmoleküle umfassen Proteine (Bezeichnung des entsprechenden Bio-Sensor-Chips: Protein-Chip) oder Glukoseverbindungen (Glukose-Chip). Die Sondenmoleküle können darüber hinaus insbesondere Markierungssubstanzen, insbesondere Farbstoffe oder redoxaktive Substanzen, sowie Linker (Molekülgruppen, die zur Verknüpfung oder Anbindung von Molekülteilen dienen) enthalten.

Unter Targets werden organische Moleküle in der Probensubstanz verstanden, insbesondere Biomoleküle, Polymere, Medikamente oder andere Wirkstoffe, die mit den Sondenmolekülen spezifisch wechselwirken können.

Im Rahmen der vorliegenden Erfindung wird ein Bio-Sensor-Chip lichtempfindlich genannt, wenn durch räumlich begrenzbare Beleuchtung eine physikalische Reaktion hervorgerufen wird, die spezifisch von der Wechselwirkung zwischen Sonden und Targets abhängt, und eine optische Adressierung der Arrayelemente (Teststellen) möglich ist.

Um die Probensubstanz dem Bio-Sensor-Chip zuführen zu können, wird eine Zuführvorrichtung eingesetzt. Dabei kommen sowohl getrennte Zuführvorrichtungen, insbesondere Spritzen, Pipetten, Schläuche, Kanülen, Röhrchen und Trichter, als auch mit dem Untersuchungssystem verbundene Vorrichtungen in Betracht. Letztere lassen sich in das Gehäuse des Untersuchungssystems integrieren und können einen Schutz des Untersuchungssystems vor Störfaktoren, wie beispielsweise Verunreinigungen, mechanische Beanspruchungen, Temperaturdifferenzen und Verdunstung, bewirken sowie eine sparsame Verwendung der Probensubstanz ermöglichen.

### Beleuchtungssystem

Im Rahmen dieser Erfindung wird unter einem Beleuchtungssystem eine oder mehrere Beleuchtungsquellen verstanden, die geeignet sind, auf einem lichtempfindlichen Bio-Sensor-Chip eine physikalische Reaktion zu initiieren. Erfindungsgemäß umfasst das Beleuchtungssystem ein Array von getrennt ansteuerbaren Beleuchtungsquellen, die so angeordnet sind, dass jeder Teststelle eines Teilarrays des Untersuchungssystems mindestens eine im wesentlichen nur sie beleuchtende Beleuchtungsquelle zugeordnet ist.

Das Beleuchtungssystem kann außerdem ein optisches Projektionssystem zum Abbilden des Arrays der Beleuchtungsquellen auf das Untersuchungssystem enthalten, derart, dass auf jede Teststelle des Teilarrays zumindest eine ihr zugeordnete Beleuchtungsquelle abbildbar ist.

Unter Projektionssystem wird jedes zur optischen Abbildung geeignete System, insbesondere bestehend aus Linsen und/oder Spiegel verstanden. Das Projektionssystem kann weitere optische Komponenten, insbesondere Filter, Blenden, Polarisatoren und Strahlteiler enthalten. Im engeren Sinne wird im Rahmen dieser Erfindung unter einem Projektionssystem ein optisches System zur Abbildung von Beleuchtungsquellen auf einen lichtempfindlichen Bio-Sensor-Chip verstanden.

Alternativ zum Vorsehen eines Projektionssystems kann das Array der Beleuchtungsquellen im wesentlichen parallel zum Untersuchungssystem und in so geringem Abstand von diesem angeordnet sein, dass die von jeder Beleuchtungsquelle emittierte Strahlung im wesentlichen nur die ihr zugeordnete Teststelle beleuchtet. In diesem Fall ist ein Projektionssystem nicht erforderlich.

Das Array aus Beleuchtungsquellen kann dabei insbesondere aus einer oder mehrere der folgenden Komponenten bestehen: Elektronenstrahlröhre (CRT - *Cathode Ray Tube*), Flüssigkristallanzeige (LCD - *Liquid Crystal Device*/*Display*) - diese bevorzugt durch eine aktive Matrix aus Dünnschichttransistoren (TFT - *Thin Film Transistor*) angesteuert, Flächenlichtmodulator (SLM *- spatial light modulator*), insbesondere ein Mikromechanisches Spiegelarray (DMD - *Digital Mirror Device -* ein Array aus einzeln ansteuerbaren Mikrospiegeln), Leuchtdioden (LED - *Light Emitting Diode*), Polymerdisplay (OLED - *Organic LED*), Elektrolumineszenzanzeige (EL), Laser - dabei besonders bevorzugt Laserdioden und Faserlaser, Plasmabildschirm (PDP - *Plasma Display*), Feldemissionsanzeige (FED - *Field Emission Display -* Array aus miniaturisierten Kathodenstrahlröhren) und Vakuumfluoreszenzanzeige (VFD - Vacuum Fluorescent Display).

Erfindungsgemäß kann das Beleuchtungssystem insbesondere auch eine Kombination aus den Komponenten "Ausleuchtungssystem" und "zweidimensionaler optischer Schalter" enthalten. Unter Ausleuchtungssystem wird hier ein optisches System zur gleichmäßigen Ausleuchtung einer bestimmten Fläche verstanden. Dieses enthält eine oder mehrere Beleuchtungsquellen und optische Komponenten, die für eine gleichmäßige Ausleuchtung sorgen, insbesondere (Spiegel-)Reflektoren, Linsen, Integratoren sowie weiterer Komponenten, wie beispielsweise Blenden zur Begrenzung des Leuchtfeldes, Filter zur spektralen Begrenzung. Unter Integratoren werden in diesem Zusammenhang optische Komponenten verstanden, die es erlauben, ein Lichtbündel zu homogenisieren, bestehend insbesondere aus Streuscheiben, beispielsweise aus Milchglas, ein Mikrolinsensystem oder einer Kombination dieser Komponenten mit einer integrierender Hohlspiegelkugel. Insbesondere kann ein Ausleuchtungssystem vom Prinzip der Mikroskop-Beleuchtungsoptik nach Köhler (Stichwort "Mikroskop", Fachlexikon Physik, Verlag Harry Deutsch, Frankfurt a. M. 1989) eingesetzt werden. Das Ausleuchtungssystem kann auch aus einem oder mehreren homogenen Leuchtfeldern, aufgebaut beispielsweise aus Glasfaserbündeln, bestehen (Hersteller: z.B. Schott-Fostec, Auburn, NY, USA).

Ein optischer Schalter ist ein optisches Element, dessen Transmission oder Reflexion von außen gesteuert werden kann. Ein zweidimensionaler optischer Schalter ist ein zweidimensionales Array aus eben solchen optischen Schaltern. Erfindungsgemäß wird dafür bevorzugt ein Flüssigkristallelement (LCD - Liquid Crystal Device) oder ein Mikromechanisches Spiegelarray (DMD, Hersteller z.B.: Texas Instruments, TX, USA) verwendet.

### Detektionssystem

Das Detektionssystem hat die Aufgabe diejenigen Teststellen, deren Sondenmoleküle mit den nachzuweisenden organischen Molekülen wechselwirken, insbesondere bei denen eine Reaktion zwischen diesen Molekülen stattgefunden hat, unter Beleuchtung zu identifizieren. Es umfasst bevorzugt einen oder mehrere Detektoren sowie Vorrichtungen zur Zuführung und Auswertung der Messsignale. Dabei wird das Detektionssystem zweckmäßig auf die Art des Ausleseverfahrens, d.h. die Art der lichtinduzierten Reaktion, des eingesetzten Untersuchungssystems abgestimmt:

Für die als Untersuchungssystem besonders bevorzugt verwendeten Bio-Sensor-Chips mit elektrischem Ausleseverfahren, insbesondere DNA-Chips mit direktem elektrischen Ausleseverfahren (insbesondere basierend auf den Patentanmeldungen DE 19921940, DE 19926457, DE 19945398) umfasst das Detektionssystem bevorzugt ein Messgerät zur Bestimmung der elektrischen Kommunikation zwischen den Teststellen und der leitfähigen Oberfläche des Bio-Sensor-Chips. Bevorzugt handelt es sich bei diesem Messgerät um ein Strom-, Ladungs-, Spannungs-, Potentialmessgerät, ein Cyclovoltametrie-Gerät, ein Amperometrie-Gerät oder ein Leitfähigkeitsmessgerät. Ein Cyclovoltametrie-Gerät ist ein Messgerät zur Aufzeichnung von Stom-Spannungskurven, wobei die Spannung zeitlich linear, periodisch verändert wird. Ein Amperometrie-Gerät ist ein Messgerät zur Aufzeichnung von Strom-Zeitkurven. Mit einem Leitfähigkeitsmessgerät lassen sich Leitfähigkeiten messen, insbesondere durch Messen des Stroms bei fester Spannung oder durch Messen der Spannung bei konstantem Strom.

Bei lichtempfindlichen Bio-Sensor-Chips mit optischem Ausleseverfahren wird die lichtinduzierte Reaktion des Chips, insbesondere Lumineszenz, optisch detektiert. Diese sind sehr verbreitet und werden beispielsweise von den Firmen Affymetrix (CA, USA), Nanogen (CA, USA), Incyte/Synteni (CA, USA) hergestellt. Werden solche optisch auslesbaren Bio-Sensor-Chips als Untersuchungssystem verwendet, umfasst das Detektionssystem zweckmäßig einen optischen Detektor zum Nachweis der lichtinduzierten Reaktion des Chips.

Der gesamte optische Aufbau, bestehend aus dem Beleuchtungssystem und gegebenenfalls einem Projektionssystem und einem optischen Detektionssystem kann zusätzlich Spiegel enthalten, um den Strahlengang zu falten und dadurch eine andere Geometrie, insbesondere eine kompaktere Bauform, zu erreichen.

Insgesamt ist auch eine Kombination aus dem im Rahmen dieser Erfindung beschriebenen Beleuchtungssystem, das ein Array getrennt ansteuerbarer Beleuchtungsquellen umfasst, und einem an sich bekannten scannenden System möglich:

Dazu wird das Beleuchtungssystem so dimensioniert, dass nur ein Teil des lichtempfindlichen Bio-Sensor-Chips beleuchtet wird. In einem ersten Schritt wird zunächst dieser Teil des Chips angeregt und ausgelesen. In einem Folgeschritt wird entweder der Chip über einen x-y-Verschiebetisch oder das Beleuchtungsfeld über bewegliche Umlenkspiegel um die Breite eines Feldes verschoben. Anschließend wird der dadurch neu ausgewählte Bereich des Chips angeregt und ausgelesen. Dieses Verfahren wird so lange wiederholt, bis der gesamte Chip ausgelesen wurde.

Bei einem Beleuchtungssystem, dass aus einem Array mit begrenzter Pixelanzahl besteht, kann durch die Kombination mit einem scannenden Verfahren eine Beleuchtung des gesamten Chips mit hoher Pixeldichte erreicht werden.

Ist andererseits die Pixelanzahl des Beleuchtungssystems groß, so ist es oft zweckmäßig jeweils mehrere Beleuchtungsquellen auf eine Teststelle abzubilden.

### Signalerfassung und Nachweisverfahren

Das vom Detektionssystem erfasste Signal wird an ein Auswertesystem, bevorzugt einen Computer, übertragen. Dadurch ist auch eine Fehlerkorrektur, insbesondere eine Korrektur von eventuellen Inhomogenitäten des Beleuchtungssystems, des Bio-Sensor-Chips oder des Detektionssystems möglich. Solche Inhomogenitäten können beispielsweise durch eine ungleichmäßige Ausleuchtung, Fertigungstoleranzen sowie durch Abschattungs- und Randeffekte verursacht sein. Zweckmäßig wird das vom Detektionssystem erfasste Signal mit dem Beleuchtungsmuster des Beleuchtungssystems und der Art sowie der Position der Teststellen auf dem Bio-Sensor-Chip korreliert.

Das Detektionssystem hat keine räumliche Auflösung, so dass alle Teststellen des lichtempfindlichen Bio-Sensor-Chips, an denen eine lichtinduzierte Reaktion stattgefunden hat, gemeinsam ausgelesen werden. Ein bevorzugtes Beispiel für diese Ausführungsform umfasst als Untersuchungssystem einen lichtempfindlichen Bio-Sensor-Chips mit elektrischem Ausleseverfahren, bei dem alle Teststellen auf einer gemeinsamen Messelektrode aufgebracht sind. Die Adressierung der einzelnen Teststellen geschieht in dieser Ausführungsform ausschließlich durch die teststellenspezifische optische Anregung.

Zum Nachweis von organischen Molekülen in einer Probensubstanz können erfindungsgemäß die im folgenden beschriebenen Verfahren eingesetzt werden:
Zunächst wird ein lichtempfindlicher Bio-Sensor-Chip als Untersuchungssystem bereit gestellt, der ein Array von Teststellen mit spezifischen Sondenmolekülen enthält. Diese Sondenmoleküle sind vorzugsweise spezifisch für die in der Probensubstanz nachzuweisenden Moleküle bzw. für die nachzuweisende Wechselwirkung zwischen diesen Sondenmolekülen und der Probensubstanz. Dazu wird ein passendes Beleuchtungssystem zur optischen Beleuchtung des lichtempfindlichen Bio-Sensor-Chips, umfassend ein Array von getrennt ansteuerbaren Beleuchtungsquellen, die so angeordnet sind, dass jeder Teststelle eines Teilarrays des Untersuchungssystems mindestens eine im wesentlichen nur sie beleuchtende Beleuchtungsquelle zugeordnet ist. Nachdem dem Untersuchungssystem eine Probensubstanz zugeführt wurde, werden die Teststellen durch Ansteuerung der ihnen jeweils zugeordneten Beleuchtungsquellen nach einem vorgewählten Schema beleuchtet. Gleichzeitig werden die Teststellen daraufhin untersucht, ob deren Sondenmoleküle mit den nachzuweisenden organischen Molekülen wechselwirken.

Diese Identifizierung dieser Wechselwirkung kann bevorzugt durch ein elektrochemisches Verfahren, insbesondere durch cyclische Voltametrie, Amperometrie, Leitfähigkeitsmessung oder eine sonstiges Strom-, Ladungs-, Spannungs- Potentialmessung erfolgen. Bei Bio-Sensor-Chips mit elektrischem Ausleseverfahren ist eine Identifizierung der Reaktion zwischen den Sondenmolekülen und den nachzuweisenden organischen Molekülen bevorzugt dadurch möglich, dass die Teststellen auf einer leitfähigen Oberfläche angeordnet sind und die Reaktion durch eine Bestimmung der elektrischen Kommunikation zwischen den Teststellen und der leitfähigen Oberfläche nachgewiesen wird. Diese Kommunikation wird wiederum bevorzugt durch ein elektrochemisches Verfahren, insbesondere durch cyclische Voltametrie, Amperometrie, Leitfähigkeitsmessung oder eine sonstiges Strom-, Ladungs-, Spannungs- Potentialmessung nachgewiesen.

Eine ebenfalls bevorzugte Identifizierung der Reaktion zwischen den Sondenmolekülen und den nachzuweisenden organischen Molekülen basiert auf einem optischen Verfahren insbesondere der Detektion von Lumineszenz.

Während bei einem scannenden Beleuchtungsverfahren nach dem Stand der Technik, beispielsweise bei Einsatz eines Laser-Scanners, typischerweise nur eine Teststelle gleichzeitig beleuchtet werden kann, erlaubt das im Rahmen dieser Erfindung beschriebene Beleuchtungssystem, mehrere Teststellen gleichzeitig zu beleuchten. Dadurch steht eine Vielzahl möglicher Beleuchtungsschemata zur Auswahl. Für das hier beschriebene Nachweisverfahren kann ein optimales Beleuchtungsschema ausgewählt werden, das auf die Gegebenheiten der Nachweisvorrichtung abgestimmt ist. Insbesondere können mehrere Teststellen, bevorzugt solche, bei denen die Wahrscheinlichkeit einer Reaktion mit den nachzuweisenden organischen Molekülen gering ist, zunächst gleichzeitig beleuchtet und ausgelesen werden. Wird innerhalb einer solchen Gruppe keine Reaktion nachgewiesen, kann eine Reaktion für die ganze Gruppe ausgeschlossen werden und auf das weitere Auslesen der individuellen Teststellen dieser Gruppe verzichtet werden. Nur wenn eine Reaktion innerhalb einer solchen Gruppe von Teststellen festgestellt wurde oder nicht zuverlässig ausgeschlossen werden konnte, ist in einem Folgeschritt eine weitere Beleuchtung und Auslesung dieser Teststellen in einer anderen Gruppierung oder individuell notwendig. Da bei einer solchen Vorgehensweise zum Teil ganze Gruppen von Teststellen gleichzeitig ausgelesen und als nicht-reagierend eingestuft werden können, kann das Ausleseverfahren insgesamt zeitlich verkürzt werden. Beispielsweise sind bei einem Chip mit N = 2ⁿ Teststellen (n ganzzahlig), bei dem nur an einer Teststelle eine Reaktion mit der Untersuchungslösung nachweisbar ist, anstatt N Ausleseschritten nur 2n+1 Ausleseschritte notwendig, wenn das folgende Ausleseverfahren angewendet wird:

Zunächst werden alle Teststellen auf einmal ausgelesen, dann jeweils eine Hälfte davon, anschließend wird die Hälfte, in der eine Reaktion nachgewiesen wurde, wiederum in zwei Hälften eingeteilt, die beide ausgelesen werden, wobei iterativ jeweils die Hälfte, in der eine Reaktion nachgewiesen wurde, weiter solange halbiert wird, bis letztendlich die Teststelle, an die eine Reaktion nachweisbar ist, eindeutig identifiziert ist. In diesem Beispiel ist dadurch auch eine Zeitersparnis um den Faktor N/(2n+1) möglich, bei einem Chip mit 1024 Testsites beispielsweise eine Zeitersparnis um den Faktor 49. Ist an mehreren Teststellen eine Reaktion nachweisbar, so ist die Zeitersparnis geringer, da sich das Iterationsverfahren verzweigen muss.

Das Beleuchtungsschema kann auch in Bezug auf die Abstände zwischen den Teststellen optimiert werden, insbesondere um Wechselwirkungen zwischen nahe beieinanderliegenden Teststellen beim Auslesen zu minimieren. Dabei wird beispielsweise in einem ersten Schritt nur jede zweite Teststelle einer Teststelle beleuchtet, während die übrigen Teststellen anschließend in einem zweiten Schritt beleuchtet werden. Bei starken Beeinflussungen und/oder geringen Abständen der Teststellen können auch größere Zwischenräume zwischen den beleuchteten Teststellen zweckmäßig sein.

Zur Steigerung der Empfindlichkeit des Ausleseverfahren, insbesondere bei einem starkem konstanten oder zeitlich variierenden Untergrundsignal, kann beim Auslesen einer Teststelle dessen Beleuchtungsstärke zeitlich variiert werden. Insbesondere ist eine Differenzmessung (Signal mit Beleuchtung - Signal ohne Beleuchtung) möglich. Darüber hinaus kann die Beleuchtung zeitlich periodisch variiert werden und das Signal (die detektierte Reaktion zwischen den Sondenmolekülen und den nachzuweisenden organischen Molekülen) frequenz- oder phasenspezifisch weiterverarbeitet werden. Dazu wird das Signal in einem Verstärker mit einem phasenempfindlichen Gleichrichter (Lock-In-Verstärker) verarbeitet, der synchron mit der pulsierenden Beleuchtung getriggert wird. Das gleichgerichtete Signal am Ausgang des Lock-In-Verstärkers ist ein dann Maß für die durch die Beleuchtung initiierte Reaktion. Durch ein solches Lock-In-Verfahren ist insbesondere eine Unterdrückung von konstanten Signalen oder von periodischen Signalen, deren Frequenz sich von derjenigen der Beleuchtungsfrequenz deutlich unterscheidet, möglich. Die beim Lock-In-Verfahren verwendete Frequenz sollte sich von in der Umgebung des Messaufbaus eventuell vorhandenen Störfrequenzen, insbesondere der Frequenz des Stromnetzes (50 bzw. 60Hz) oder ganzzahligen Vielfachen davon, unterscheiden, muss kleiner sein als die Grenzfrequenzen des verwendeten Beleuchtungssystems und Detektionssystems und liegt bevorzugt im Bereich von 1 Hz bis 100 MHz liegen, wobei die obere Grenzfrequenz in miniaturisierten Auslesesystemen auch höher sein kann. Besonders bevorzugt werden Frequenzen im kHz-Bereich verwendet, beispielsweise 3 kHz.

Bei periodischer Beleuchtung der Teststellen können auch mehrere Teststellen gleichzeitig ausgelesen werden, indem verschiedene Teststellen mit unterschiedlichen Frequenzen oder Phasen beleuchtet werden und die Identifizierung der Reaktion zwischen den Sondenmolekülen einer Teststelle und den nachzuweisenden organischen Molekülen mit Hilfe frequenz- oder phasenspezifischer Signalverarbeitung erfolgt.

Neben einer Steigerung der Empfindlichkeit und/oder einer Verkürzung der Messzeit ist ein weiterer Vorteil der periodischen Beleuchtung und Auslesung der Teststellen eine bessere Regenerationsfähigkeit des Untersuchungssystems. Beispielsweise können durch die optische Anregung erzeugte, unerwünschte Zwischenzustände, insbesondere Triplett-Zustände, der Sonden, Targets oder anderer Moleküle in der Umgebung der gerade adressierten Teststelle in der Dunkelphase in den Grundzustand relaxieren. Eine Regeneration kann auch darin bestehen, dass eventuelle, durch das Ausleseverfahren insbesondere in der Umgebung der Teststelle umgesetzte Substanzen, insbesondere Ionen bei einem elektrischen Ausleseverfahren, abtransportiert bzw. nachgeliefert werden.

### Kurze Beschreibung der Zeichnungen

Die Erfindung soll nachfolgend anhand von Ausführungsbeispielen im Zusammenhang mit den Zeichnungen näher erläutert werden. Es zeigen
- Fig. 1: Eine schematische, perspektivische Darstellung der prinzipiellen Zuordnung von Beleuchtungsquellen zu Teststellen auf dem Untersuchungssystem.
- Fig.2: Eine schematische Darstellung des prinzipiellen Aufbaus der erfindungsgemäßen Vorrichtung.
- Fig. 3: Schematische Darstellungen von Ausführungsbeispielen gemäß der Erfindung, wobei für das Beleuchtungssystem eine Flüssigkristallanzeige (LCD) verwendet wird (Ausführungsform 1).
Fig. 3A bis 3C zeigen Vorrichtungen, bei denen neben einer Beleuchtungsoptik ein Projektionssystem zum Einsatz kommt (Ausführungsform 1a).
Fig. 3D und 3E zeigen einfacher aufgebaute Vorrichtungen ohne Projektionssystem, bei denen der Bio-Sensor-Chip sehr nahe am Flüssigkristall angeordnet ist (Ausführungsform 1b).

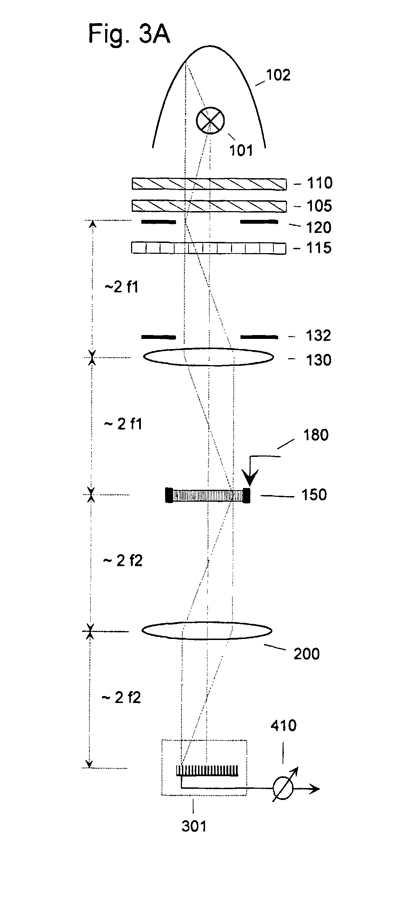

- Fig. 3A: Ein Ausführungsbeispiel gemäß der Erfindung mit LCD, Projektionssystem und elektrischer Detektion.

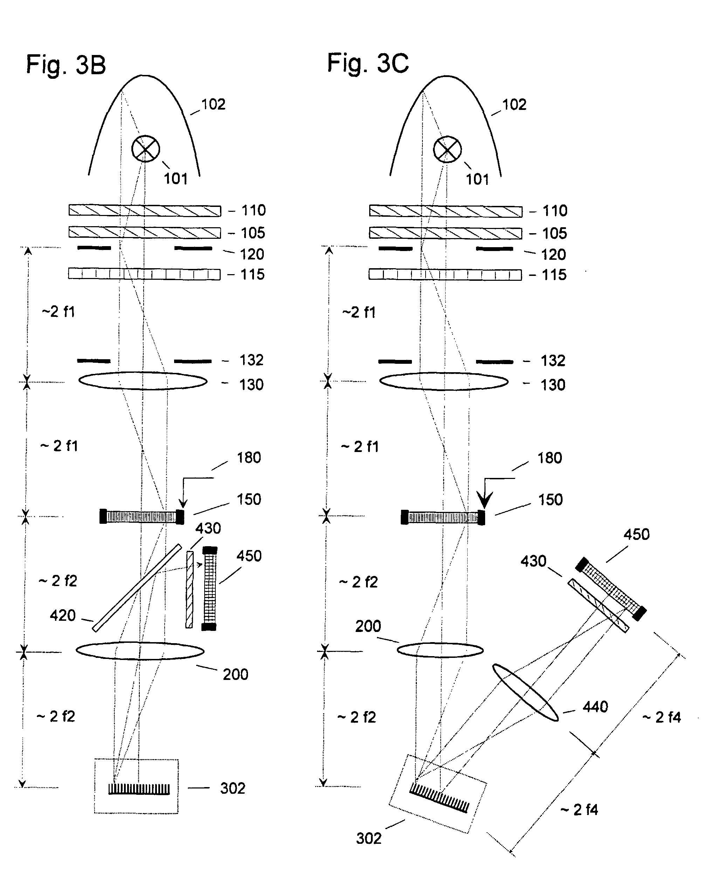

- Fig. 3B: Ein Ausführungsbeispiel gemäß der Erfindung mit LCD, Projektionssystem und optischer Detektion mittels Strahlteiler.
- Fig. 3C: Ein Ausführungsbeispiel gemäß der Erfindung mit LCD, Projektionssystem und optischer Detektion (nicht-kollinear).

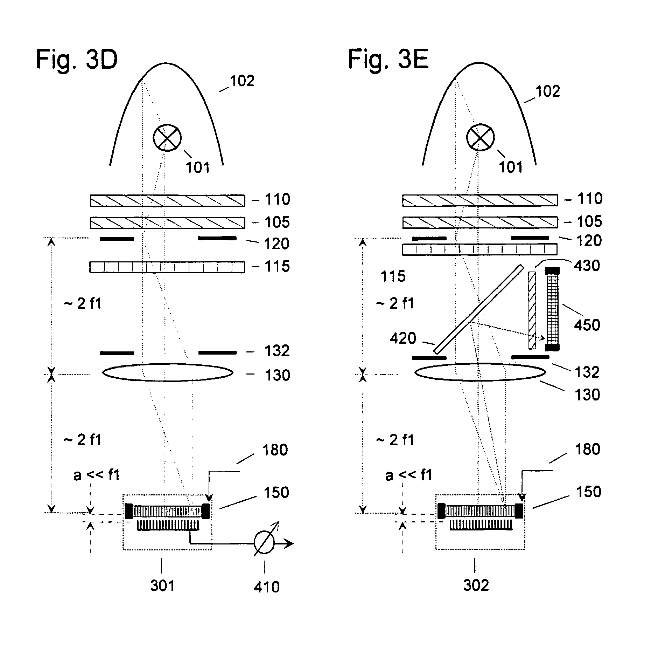

- Fig. 3D: Ein Ausführungsbeispiel gemäß der Erfindung ohne Projektionssystem mit LCD und elektrischer Detektion.
- Fig. 3E: Ein Ausführungsbeispiel gemäß der Erfindung ohne Projektionssystem mit LCD und optischer Detektion mittels Strahlteiler.

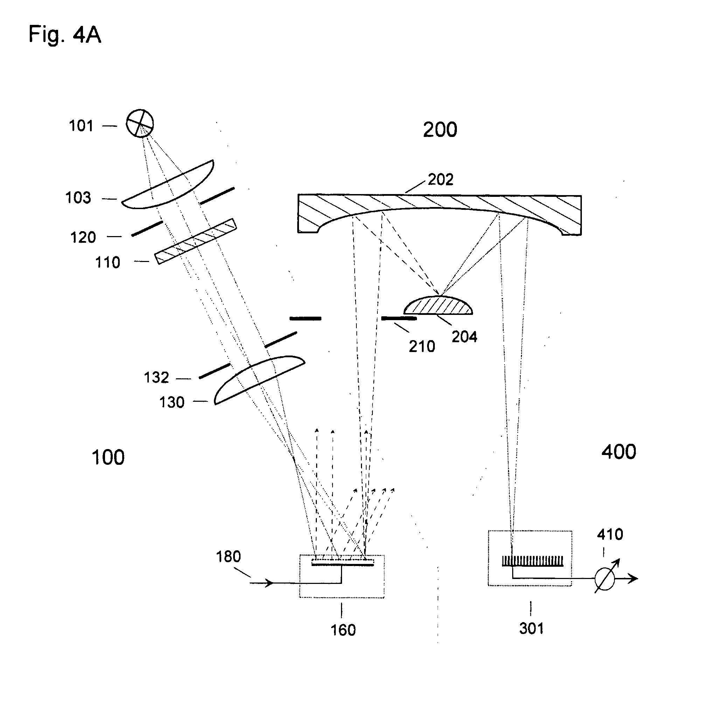

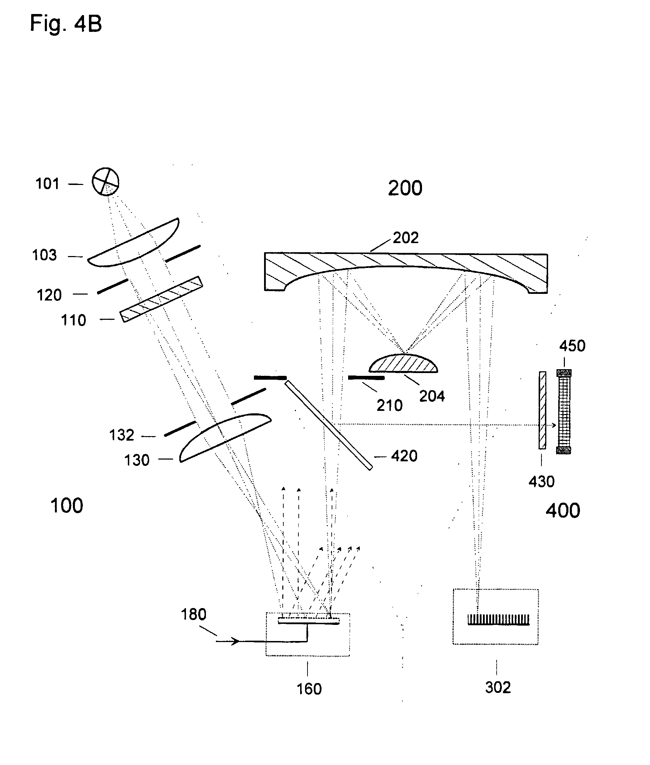

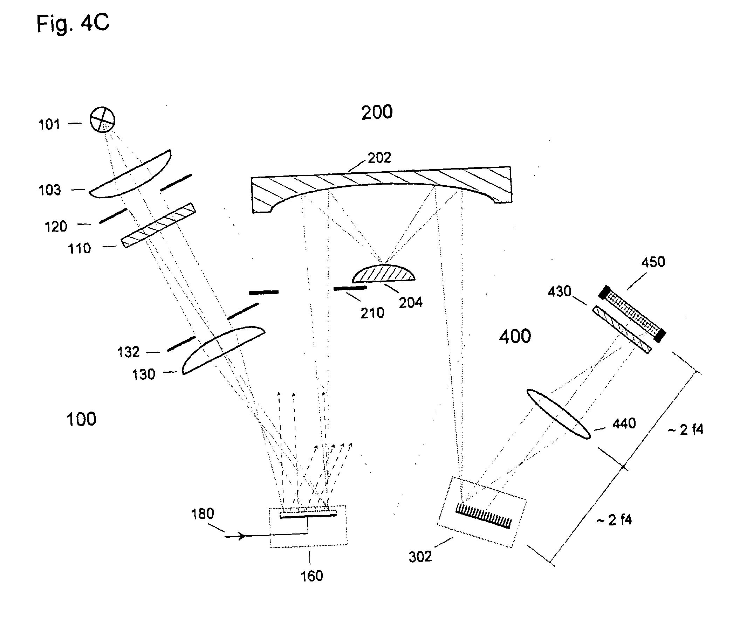

- Fig. 4: Schematische Darstellungen von Ausführungsbeispielen gemäß der Erfindung, wobei für das Beleuchtungssystem ein Mikromechanisches Spiegelarray (DMD) verwendet wird (Ausführungsform 2).
- Fig. 4A: Ein Ausführungsbeispiel gemäß der Erfindung mit DMD, Projektionssystem und elektrischer Detektion.
- Fig. 4B: Ein Ausführungsbeispiel gemäß der Erfindung mit DMD, Projektionssystem und optischer Detektion mittels Strahlteiler.
- Fig. 4C: Ein Ausführungsbeispiel gemäß der Erfindung mit DMD, Projektionssystem und optischer Detektion (nicht-kollinear).

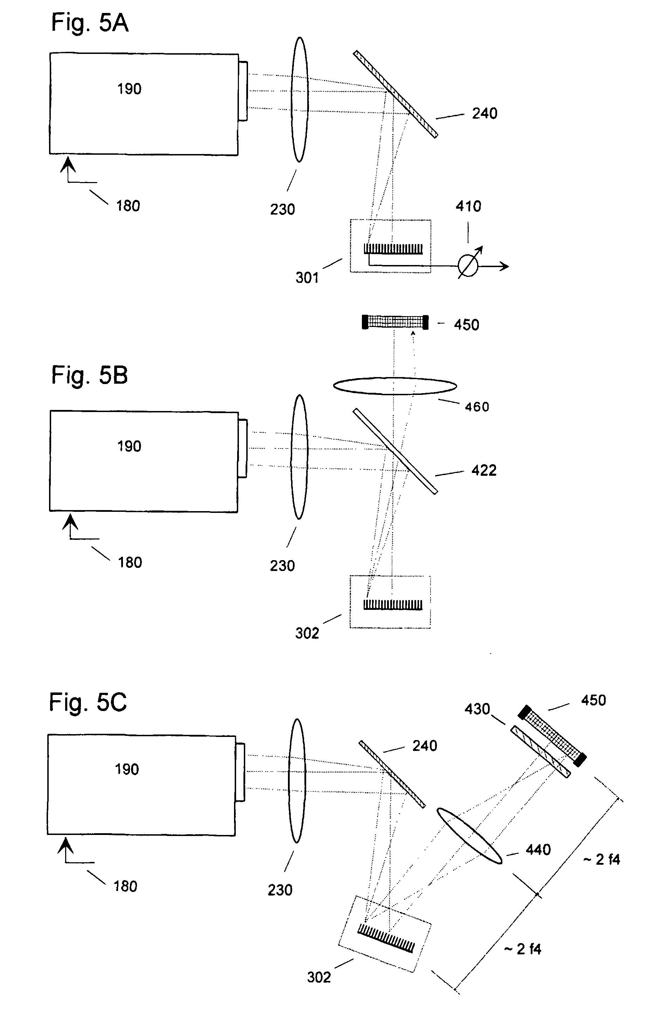

- Fig. 5: Schematische Darstellungen von Ausführungsbeispielen gemäß der Erfindung, wobei für das Beleuchtungssystem ein Videoprojektor verwendet wird (Ausführungsform 3).
- Fig. 5A: Ein Ausführungsbeispiel gemäß der Erfindung mit Videoprojektor, Projektionssystem und elektrischer Detektion.
- Fig. 5B: Ein Ausführungsbeispiel gemäß der Erfindung mit Videoprojektor, Projektionssystem und optischer Detektion mittels Strahlteiler.
- Fig. 5C: Ein Ausführungsbeispiel gemäß der Erfindung mit Videoprojektor, Projektionssystem und optischer Detektion (nicht-kollinear).

### Wege zur Ausführung der Erfindung

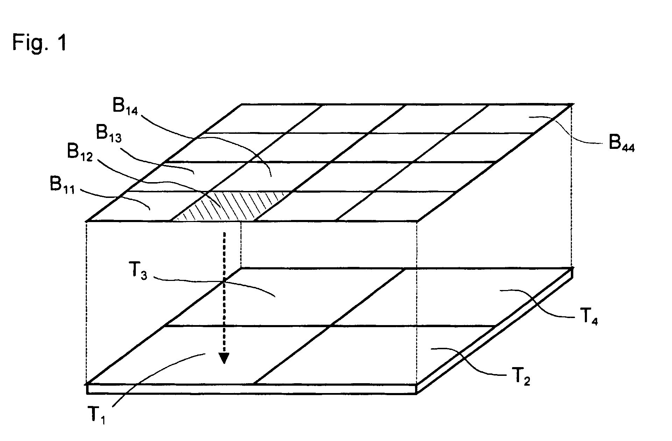

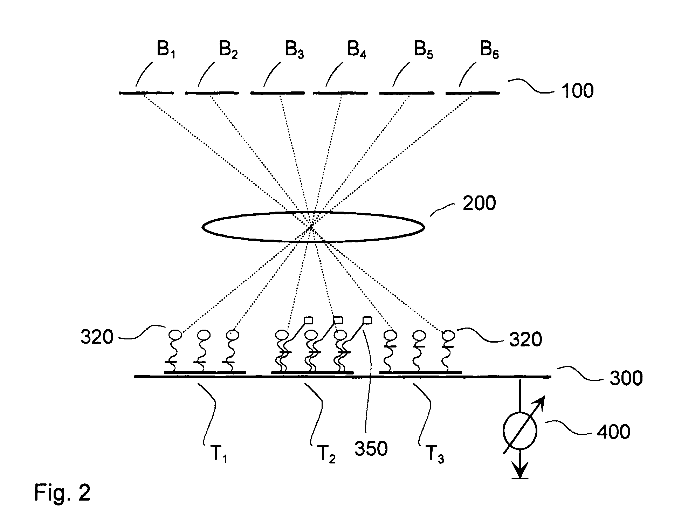

Das Arbeitsprinzip ist zunächst anhand der Figuren 1 und 2 erläutert. Das Untersuchungssystem der Ausführungsform von Fig. 1 umfasst ein Array von Teststellen (T₁ - T₄), wobei jede Teststelle spezifische Sondenmoleküle aufweist. Um organische Moleküle (Targets) in einer Untersuchungslösung nachzuweisen, werden diese dem Untersuchungssystem zugeführt. Das Untersuchungssystem hat die Eigenschaft, bei Beleuchtung einer beliebigen Teststelle eine physikalische Reaktion zu zeigen, die spezifisch von der Wechselwirkung zwischen den Targets und den Sonden dieser Teststelle abhängt. Ein wesentliches Merkmal dieser Erfindung ist die Beleuchtung der Teststellen des Untersuchungssystems T₁ - T₄ mit einem Beleuchtungssystem, das hier ein Array von Beleuchtungsquellen B₁₁ - B₄₄ umfasst. Dabei wird jeder Teststelle mindestens eine, im wesentlichen nur sie beleuchtende Beleuchtungsquelle zugeordnet. In der Ausführungsform von Fig. 1 sind je vier Beleuchtungsquellen einer Teststelle zugeordnet, beispielsweise die Quellen B₁₁, B₁₂, B₁₃ und B₁₄ der Teststelle T₁. Exemplarisch ist deren Beleuchtung durch die aktivierte Beleuchtungsquelle B₁₂ dargestellt.

Fig. 2 zeigt in schematischer Darstellung das Grundprinzip einer erfindungsgemäßen Vorrichtung, enthaltend ein Beleuchtungssystem 100, Projektionssystem 200, Untersuchungssystem 300 und Detektionssystem 400. Das Beleuchtungssystem 100 besteht aus den einzeln ansteuerbaren Beleuchtungsquellen B₁ - B₆, die über das Projektionssystem 200, auf die Teststellen T₁ - T₃ des Untersuchungssystems 300 projiziert werden können. Die Teststellen T₁ - T₃ umfassen spezifische Sondenmoleküle 320, die mit Targets 350 aus der Untersuchungslösung spezifisch wechselwirken können. Exemplarisch sind Targets 350 gezeigt, die spezifisch mit den Sonden der Teststelle T₂ reagieren. Bei Beleuchtung mit einer der dieser Teststelle zugeordneten Beleuchtungsquellen B₃ oder B₄ wird die physikalische Reaktion des Untersuchungssystems 300 mit dem Detektionssystem 400 nachgewiesen. Diese physikalische Reaktion ist beispielsweise ein erhöhter Stromfluss durch die Teststelle T₂, bedingt durch eine erhöhte Leitfähigkeit des konkreten Systems Sonden+Targets verglichen mit den nicht reagierten Sondenmolekülen. Dabei befinden sich die Teststellen T₁ - T₃ auf einer leitfähigen Oberfläche des Untersuchungssystems 300. Der durch die Beleuchtung induzierte Stromfluss von den angebundenen Molekülen über die leitfähige Oberfläche zu Masse wird über ein Strommessgerät 400 nachgewiesen. Dabei kann die Reaktion mehrerer Teststellen T₁ - T₃ gemeinsam (wie in Fig. 2) oder jeder Teststelle einzeln, beispielsweise über einzeln ansteuerbare Elektroden, detektiert werden. Werden mehrere Teststellen, wie T₁ - T₃ in Fig. 2, über eine gemeinsame Elektrode ausgelesen, kann eine Teststelle, an der eine Reaktion der Sonden- mit den Targetmolekülen stattgefunden hat, durch eine selektive Beleuchtung identifiziert werden:

In der Situation von Fig. 2 ergibt sich beispielsweise ein Stromfluss am Messgerät 400 bei Beleuchtung des Untersuchungssystems durch die Beleuchtungsquellen B₃ oder B₄. Sind dagegen nur die Quellen B₁ und B₂ oder B₅ und B₆ aktiv, ergibt sich mangels erfolgter Reaktion an den Teststellen T₃ bzw. T₁ kein Stromfluss.

Bei vorgegebener Zuordnung von Beleuchtungsquellen und Teststellen kann auf diese Weise auch bei Auslesen des Untersuchungssystems mit einer gemeinsamen Elektrode eine ortsaufgelöste Detektion erfolgen.

Eine Aufbringung einzelner Teststellen oder Gruppen von Teststellen auf getrennte Elektroden kann trotzdem sinnvoll sein, beispielsweise um eine bessere Selektivität und ein besseres Signal-Rausch-Verhältnis zu erreichen oder die Wechselwirkung von Sonden- und Targetmolekülen durch räumlich begrenzte elektrische Felder beeinflussen zu können.

### Bevorzugte Ausführungsformen:

Einige spezielle Ausführungsformen der Erfindung werden nachfolgend mit Bezug auf die Figuren 3A bis 5C näher beschrieben. Dabei können Einzelelemente der dargestellten Vorrichtungen auch auf andere Weise miteinander kombiniert werden. Prinzipiell lassen sich unterschiedliche Beleuchtungssysteme 100 mit verschiedenen Projektionssystemen 200 und Untersuchungssystemen 300 sowie für das zugehörige Ausleseverfahren geeigneten Detektionssystemen 400 kombinieren. Darüber hinaus lassen sich insbesondere zum Aufbau eines Beleuchtungssystems 100 unterschiedliche Ausleuchtungssysteme (bestehend aus den Komponenten mit den Bezugszeichen 101 - 149) mit verschiedenen zweidimensionalen optischen Schaltern (150, 160) kombinieren. Außerdem lassen sich unterschiedliche Detektoren einsetzen.

### Ausführungsformen vom Typ 1: Beleuchtungssystem mit Flüssigkristall (LCD)

Eine erste Gruppe von Ausführungsformen (Fig. 3A - 3E) zeichnet sich dadurch aus, dass als zweidimensionaler optischer Schalter ein Flüssigkristall-Element (LCD) 150, als Beispiel für einen transmittierenden optischen Schalter, eingesetzt wird.

Das Ausleuchtungssystem (101 - 132) ist an die Mikroskop-Beleuchtungsoptik nach Köhler angelehnt. Es besteht aus einer Lampe 101 mit Spiegelreflektor 102, einem optionalen Integrator 105, einem Kondensor 130 sowie Filtern 110 und Blenden 120/132. Die Lampe 101 wird in Kombination mit einem oder mehreren spektralen Filtern 110 so gewählt, dass die emittierte Strahlung spektral auf den verwendeten Bio-Sensor-Chip 301/302 abgestimmt ist. Insbesondere können dafür Glühlampen, bevorzugt Halogenlampen, oder Bogenlampen eingesetzt werden. Der Reflektor 102, bevorzugt mit parabolischer Form, ist so positioniert, dass das von der Lampe emittierte Licht parallel auf den Integrator 105 auftritt. Dieser hat die Aufgabe den Lichtstrahl zu homogenisieren und für eine gleichmäßige Ausleuchtung zu sorgen. Insbesondere können dafür Streuscheiben, beispielsweise aus Milchglas, oder ein Mikrolinsensystem verwendet werden. Die dicht hinter dem Integrator 105 angebrachte Leuchtfeldblende 120 begrenzt die Strahlung auf eine Fläche, die gerade groß genug ist, um den Flüssigkristall 150 auszuleuchten. Der Kondensor 130 besteht aus einer Sammellinse oder auch einem Linsensystem und hat die Aufgabe, das von der Leuchtfeldblende 120 begrenzte homogene Strahlungsfeld auf den Flüssigkristall 150 abzubilden. Er hat eine Brennweite f1 bevorzugt im Bereich 10 bis 300 mm, im Ausführungsbeispiel von 100 mm, und ist so positioniert, dass die Leuchtfeldblende 120 scharf auf den Flüssigkristall 150 abgebildet wird. Beispielsweise wird der Kondensor 130 genau in der Mitte zwischen Leuchtfeldblende und Flüssigkristall jeweils im Abstand 2 x f1 angeordnet, wenn die laterale Ausdehnung von Leuchtfeldblende 120 und Flüssigkristall 150 gleich groß und damit eine Vergrößerung V=1 gewählt wird. Bei unterschiedlichen Größen sind Vergrößerungen ungleich eins, bevorzugt im Bereich V=1/5 bis 5, mit anderen Abständen gemäß den Abbildungsgleichungen des Kondensors 130 möglich.

Da das Flüssigkristall-Element 150 nur polarisiertes Licht transmittert, kann bereits das Ausleuchtungssystem ein Polarisationsfilter 115, vorzugsweise ein verlustarmer Polarisationsfilter (*polarization recovery plate*), enthalten, bevorzugt in der Nähe der Leuchtfeldblende 120 angeordnet. Der verlustarmer Polarisationsfilter lässt eine Polarisationskomponente des einfallenden Lichts durch und wirkt auf dazu senkrechte reflektierte Polarisationskomponenten, indem er deren Polarisationsrichtungen dreht und sie wieder in den Strahlengang einfügt. Dadurch treten bei der Polarisierung insgesamt weniger Verluste auf, als bei einem einfachen Polarisator.

Direkt vor der Kondensorlinse 130 ist eine weitere Blende - die Kondensorblende 132 - zur Einstellung der Lichtintensität des Ausleuchtungssystems angebracht.

Als zweidimensionaler Schalter wird bei diesen Ausführungsformen ein in Transmission zu betreibender zweidimensionaler optischer Schalter, exemplarisch ein Flüssigkristallelement (LCD) 150, eingesetzt. Er hat in beiden Dimensionen eine Pixelanzahl, die mindestens der Anzahl der Matrixelemente (Teststellen) auf dem auszulesenden Bio-Sensor-Chip, bevorzugt mindestens fünfmal so vielen, entspricht. Durch eine Computeransteuerung 180 werden die einzelnen Pixel und damit letztendlich die Helligkeit des Beleuchtungssystems an der entsprechenden Position gesteuert. Bis zu diesem Punkt sind die Ausführungsformen 1a und 1b identisch.

Die Ausführungsformen von Fig. 3A - 3C enthalten zusätzlich ein Projektionssystem 200, das das beleuchtete LCD 150 auf den Bio-Sensor-Chip 301/302 projiziert. Das Projektionssystem 200 besteht aus einer Sammellinse oder einem Linsensystem. Es wird in einer dem Fachmann geläufigen Weise so dimensioniert und positioniert, dass das LCD 150 scharf auf den Bio-Sensor-Chip 301/302 abgebildet und eine Vergrößerung gewählt wird, die gerade eine vollständige Beleuchtung des Chips 301/302 ermöglicht. Beispielsweise wird bei einem Bio-Sensor-Chip 301/302 der Größe 20 mm x 20 mm und einem gleich großen LCD 150 die Vergrößerung eins gewählt, indem die Projektionsoptik 200 mit der Brennweite f2, bevorzugt im Bereich 10 bis 300 mm, im Ausführungsbeispiel 100 mm, genau in der Mitte zwischen LCD 150 und Chip 301/302 jeweils im Abstand 2 x f2 angeordnet wird. Dabei wird der Abstand zwischen Chip 301/302 und Projektionsoptik 200 und damit auch f2 ausreichend groß gewählt, so dass zum einen der Bio-Sensor-Chip 301/302 mit seiner Halterung und eventuell damit verbundene Probenzuführungssystem Platz unter der Projektionsoptik 200 findet und leicht ausgewechselt werden kann. Zum anderen wird der Abstand bevorzugt deutlich größer gewählt als die seitliche Ausdehnung des Chips 301/302, damit der Einfallswinkel der Beleuchtungsstahlen an verschiedenen Positionen auf dem Chip und damit die Beleuchtungsintensität auf jeder Flächeneinheit möglichst gleich ist. Unterschiede in der Beleuchtungsintensität können jedoch auch durch die Auswertesoftware korrigiert werden.

Die Auslesung des Bio-Sensor-Chips 301/302 hängt vom verwendeten Chip-Typ ab. Bei den besonders bevorzugten elektrisch auslesbaren Bio-Sensor-Chips 301 (Fig. 3A), werden elektrische Signale gemessen und mit dem Ansteuerungsmuster des LCD 150 durch die Auswertesoftware korreliert. In diesem Fall sind keine weiteren optischen Elemente notwendig.

Ein Bio-Sensor-Chip mit optischem Ausleseverfahren (Lumineszenz-Chips) 302, der mit räumlich auflösendem Detektionssystem nicht Teil der Erfindung ist, wird mit Bezug auf die Fig. 3B und 3C erläutert. Das optische Detektionssystem (420 - 450) besteht zumindest aus einem optischen Detektor 450, vorzugsweise eine CCD-Kamera bzw. intensified CCD-Kamera (Hersteller: z.B. Hamamatsu Photonics, Herrsching, D) oder einer CMOS-Kamera (Hersteller: z.B. Frauenhofer-Institut dür Mikroelektronische Schaltungen und Systeme, Duisburg, D), im gezeigten Beispiel eine CCD-Kamera. Vor dem Detektor 450 wird ein optischer Filter 430 angebracht, der spezifisch für die Lumineszenzwellenlänge des Bio-Sensor-Chips 302 ist und Streulicht unterdrückt.

Eine Ausgestaltung (Fig. 3B) enthält außerdem einen Strahlteiler 420 zwischen LCD 150 und Projektionsoptik 200, der die vom Bio-Sensor-Chip 302 emittierte Strahlung mit Hilfe der Projektionsoptik 200 auf den Detektor 450 projiziert. Haben LCD 150 und Detektor 450 in etwa die gleiche Größe, so ist dazu keine weitere Detektionsoptik erforderlich. Die CCD-Kamera 450 wird in diesem Fall direkt im über den Strahlteiler 420 umgelenkten Fokus der Projektionsoptik 200 positioniert. Bei unterschiedlichen Größen von LCD 150 und Detektor 450 wird eine Anpassung an die Detektorfläche durch eine zusätzliche Detektionsoptik - entweder in der CCD-Kamera 450 enthalten oder als externes optisches System (440, siehe unten) - mit der entsprechenden Vergrößerung gewährleistet. Eine Steigerung der Empfindlichkeit und eine Streulichtunterdrückung kann durch Verwendung eines dicroitischen Strahlteilers 420 erreicht werden, der bei der Wellenlänge des Anregungslichts eine größere Transmission hat als bei der Wellenlänge des zu detektierenden Lichts. Insgesamt ist auch eine Vertauschung der Zuordnung "Anregung = transmittiertes Strahl" und "Detektion = reflektierter Strahl" möglich.

Eine weitere bevorzugte Ausführungsform (Fig. 3C) basiert auf einer nicht-kollinearen Anordnung von Anregungs- und Detektionsstrahlengang (off-axis Anordnung). Dadurch ist es möglich, eine separate Detektionsoptik 440 getrennt von der Projektionsoptik 200 anzubringen. Diese Optik 440 besteht aus einer Sammellinse, einem Linsensystem oder einer Spiegeloptik und ist so dimensioniert, dass der Bio-Sensor-Chip 302 scharf und in voller Größe auf den Detektor 450 abgebildet wird. Durch einen optionalen Ablenkspiegel kann der Anregungs- und der Detektionsstrahlengang räumlich weiter voneinander getrennt werden.

Bei den Ausführungsformen von Fig. 3D und 3E wird auf das Projektionssystem 200 verzichtet. Dies ist möglich, wenn der Flüssigkristall 150 mindestens die gleiche Größe und Auflösung wie der Bio-Sensor-Chip 301/302 hat. Der Flüssigkristall 150 wird in diesem Fall direkt, parallel über dem Bio-Sensor-Chip 301/302 angeordnet, so dass das Beleuchtungsmuster des LCD 150 direkt auf den Chip projiziert wird. Um eine Anregung von benachbarten Teststellen (Tᵢ) auf dem Chip 301/302 auszuschließen, ist in dieser Ausführungsform der Abstand a zwischen LCD 150 und Chip kleiner als der Abstand der Teststellen auf dem Chip geteilt durch den Sinus des Austrittwinkel des Anregungslichts aus dem LCD.

Diese Ausführungsform kann bevorzugt für elektrisch auslesbaren Bio-Sensor-Chips 301 eingesetzt werden, bei dem zum Nachweis der Reaktion des Chips keine weiteren optischen Komponenten (420 - 450) erforderlich sind (Fig. 3D). Bei Chips mit optischem Auslesverfahren 302 ist prinzipiell eine Detektion der Lumineszenz über einen zwischen Kondensor 130 und Leuchtfeldblende 120 integrierten Strahlteiler 420 (Fig. 3E) oder mit einer nicht-kollinearen Anordnung von Anregungsund Detektionsstrahlengang möglich - in analoger Weise wie bei der Ausführungsform 1a beschrieben. Dabei müssen jedoch Signalverluste aufgrund des doppelten Durchgangs durch den Flüssigkristall (Anregungs- und Lumineszenzlicht), insbesondere beim schrägen Durchgang der nicht-kollinearen Anordung, in Kauf genommen werden. Ein Vorteil bei dieser Variante ist das höhere Kontrastverhältnis aufgrund des doppelten Durchgangs durch das LCD.

### Ausführungsformen vom Typ 2: Beleuchtungssystem mit mikromechanischem Spiegelarray (DMD)

Die Ausführungsformen vom Typ 2 (Fig. 4A - 4C) zeichnen sich dadurch aus, dass als zweidimensionaler optischer Schalter ein Mikromechanisches Spiegelarray (DMD) 160, als Beispiel für einen reflektierenden optischen Schalter, eingesetzt wird. Das DMD ist ein Array aus einzeln ansteuerbaren Mikrospiegeln, wie es insbesondere von der Firma Texas Instruments oder vom Frauenhofer-Institut für mikroelektronische Schaltungen und Systeme mittels Silizium-Chiptechnologie hergestellt wird. Durch den Einsatz eines DMD kann eine große Beleuchtungsstärke bei einem hohen Füllfaktor und einem großen Kontrastverhältnis erreicht werden. Der Füllfaktor gibt bei einem zweidimensionalen optischen Schalter das Verhältnis der effektiv schaltbaren Fläche zur Gesamtfläche an. Aufgrund von Zwischenräumen zwischen den einzelnen Matrixelementen ist der Füllfaktor generell kleiner als 1, bei einem DMD jedoch im allgemeinen größer als bei einem LCD.

Das Ausleuchtungssystem (101 - 132) besteht aus einer Lampe 101 mit Kollektorlinse 103, einem optionalen Integrator (105), einem Kondensor 130 sowie Filtern 110 und Blenden 120 und 132. Wie bei den Ausführungsformen vom Typ 1 wird die Lampe 101 in Kombination mit einem oder mehreren spektralen Filtern 110 so gewählt, dass die emittierte Strahlung spektral auf den verwendeten Bio-Sensor-Chip 301/302 abgestimmt ist. Die Kollektorlinse 103 mit einer Brennweite im Bereich 20 mm bis 200 mm, im Ausführungsbeispiel 80 mm, ist im Abstand ihrer Brennweite von der Lampe 101 entfernt, so dass ein paralleles Lichtstrahlenbündel aus ihr austritt. Dieses Strahlenbündel wird durch die Leuchtfeldblende 120 räumlich begrenzt und kann durch einen vor ihr angeordneten Integrator (105) zusätzlich homogenisiert werden.

Wie bei den Ausführungsformen vom Typ 1 besteht der Kondensor 130 aus einer Sammellinse oder auch einem Linsensystem und hat die Aufgabe, das von der Leuchtfeldblende 120 begrenzte homogene Strahlungsfeld auf das DMD 160 abzubilden. Direkt vor der Kondensorlinse 130 ist eine weitere Blende - die Kondensorblende 132 - angebracht. Damit ist eine Einstellung der Lichtintensität des Ausleuchtungssystems möglich.

Als zweidimensionaler Schalter wird bei den Ausführungsformen vom Typ 2 ein in Reflexion zu betreibender zweidimensionaler optischer Schalter, exemplarisch ein Mikromechanisches Spiegelarray (DMD, Hersteller: Texas Instruments) 160, eingesetzt. Er hat in beiden Dimensionen eine Anzahl von Mikrospiegeln, die mindestens der Anzahl der Teststellen (Tᵢ) auf dem auszulesenden Bio-Sensor-Chip 301/302, bevorzugt mindestens fünfmal so vielen, entspricht. Durch eine Computeransteuerung 180 können die einzelnen Mikrospiegel verkippt und damit letztendlich die Helligkeit des Beleuchtungssystems 100 an der entsprechenden Position gesteuert werden.

Im Gegensatz zu den Ausführungsformen aus Fig. 3D,E ist bei den Ausführungsformen vom Typ 2 auf jeden Fall ein Projektionssystem 200 erforderlich, das das beleuchtete DMD 160 auf den Bio-Sensor-Chip 301/302 projiziert. Alternativ zu dem in Fig. 3A-C dargestellten Projektionssystem 200 ist generell ein Projektionssystem bestehend aus Spiegeloptiken 202, 204 möglich. Das hier einsetzbare Projektionssystem nach Offner (Optical Engineering, 14 (1975) S. 130-132) besteht aus je einem konkaven 202 und konvexen 204 Spiegel und bildet das DMD 160 auf den Bio-Sensor-Chip 301/302 im Verhältnis 1:1 ab. Der notwendige Vergrößerungsfaktor entspricht dem Größenverhältnis zwischen Bio-Sensor-Chip 301/302 und DMD 160. Aufgrund der begrenzten numerischen Apertur des Projektionssystems 200 (z.B. NA ∼ 0,08), gelangt nur das von den Mikrospiegeln des DMD einer bestimmten Orientierung (aktive Stellung) reflektierte Licht auf den Bio-Sensor-Chip. Je stärker ein Mikrospiegel verkippt wird (passive Stellung), desto mehr wird das Anregungslicht dieses Pixels ausgeblendet. Um die Ausblendung definiert zu gewährleisten, enthält das Projektionssystem eine Projektionsblende 210.

Die Auslesemöglichkeiten des Bio-Sensor-Chips bestehen wie bei den Ausführungsformen von Fig. 3A-C. Insbesondere sind Ausführungsformen mit elektrischem Ausleseverfahren (Fig. 4A) und optischem Ausleseverfahren mit Strahlteiler (Fig. 4B) oder nicht-kollinear (Fig. 4C) möglich. Im Fall der optischen Auslesung über einen Strahlteiler 420 (Fig. 4B) wird dieser zwischen DMD 160 und Projektionssystem 200 integriert.

### Ausführungsformen vom Typ 3: Beleuchtungssystem mit Videoprojektor

Für die Ausführungsformen vom Typ 3 (Fig. 5A - 5C) wird ein Videoprojektor 190 als Beleuchtungssystem eingesetzt. Dieser kann als Komplettsystem gekauft werden, ein LCD (Hersteller: z.B. Philipps; Sanyo), ein DMD (Hersteller: z.B. Digital Projection, Machester, UK) enthalten oder nach einem anderen Prinzip arbeiten. Bei der Auswahl sind folgende Kriterien zu beachten: Der Videoprojektor der Ausführungsformen 3 hat eine Pixelanzahl (im Ausführungsbeispiel 800 x 600), die mindestens der Anzahl der Teststellen auf dem Bio-Sensor-Chip entspricht, ein hohes Kontrastverhältnis (größer 100:1), ist auf eine Fläche der Größe des Bio-Sensor-Chips (20 mm x 20 mm) fokussierbar, weist eine ausreichende räumliche und zeitliche Stabilität sowie Homogenität und eine ausreichende Beleuchtungsstärke im für den Bio-Sensor-Chip geeigneten Spektralbereich (insbesondere bei 400 nm bis 1000 nm) auf und ist durch einen Computer ansteuerbar.

Das vom Videoprojektor ausgehende Bild wird über einen Spiegel 240 (Fig. 5A, 5C) oder Strahlteiler 422 (Fig. 5B) auf den Bio-Sensor-Chip 301/302 projiziert. Ein zusätzliches Projektionssystem 230 ist erforderlich, wenn mit dem Videoprojektor selbst keine Projektion des Bildes auf die Größe des Bio-Sensor-Chips möglich ist - was bei handelsüblichen Geräten oft der Fall ist. Für Bio-Sensor-Chips mit geringer Dichte der Testellen (Abstand benachbarter Teststellen mindestens 0,1 mm) kann das Projektionssystem 230 durch eine einzelne Sammellinse (f3 = 10 .. 300 mm) realisiert werden (Fig. 3). Diese erzeugt ein scharfes Bild, das - im Gegensatz zum üblichen Gebrauch eines Videoprojektors - nahe (< 500 mm) am Projektor 190 liegt und klein (20 mm x 27 mm) ist. An der Stelle dieses Bildes kann der Bio-Sensor-Chip 301/302 positioniert und optisch angeregt werden.

Ein kleineres Beleuchtungsfeld und damit eine höhere Pixeldichte lässt sich in an sich bekannter Weise durch zwei optische Systeme erreichen. Mit einem ersten System wird nahe (< 500 mm) am Projektor ein Zwischenbild (Kantenlänge maximal 100 mm) erzeugt. Dieses wird über eine zweites System auf die Größe des Bio-Sensor-Chips 301/302 verkleinert.

Eine weitere Variante der Ausführungsformen vom Typ 3 besteht darin, das Projektionslinsensystem des Videoprojektors 190 auszutauschen bzw. kundenspezifisch anzupassen, so dass eine Projektion auf die Größe des Bio-Sensor-Chips 301/302 möglich ist.

Bei den Ausführungsformen mit Videoprojektor bestehen ebenfalls die Auslesemöglichkeiten des Bio-Sensor-Chips 301/302 wie bei den Ausführungsformen von Fig. 3A-C. Insbesondere sind Ausführungsformen mit elektrischem Ausleseverfahren (Fig. 5A) und optischem Ausleseverfahren mit Strahlteiler (Fig. 5B) oder nicht-kollinear (Fig. 5C) möglich. Im Fall der optischen Auslesung über einen Strahlteiler 422 (Fig. 5B) wird in der Ausführungsform mit Videoprojektor 190 eine Detektionsoptik 460 eingesetzt um den Bio-Sensor-Chip 302 auf den optischen Detektor 450 abzubilden.

### Erhöhung der Auflösung bei allen drei Ausführungsformen

Ist die Zahl der Pixel des zweidimensionalen optischen Schalters 150/160 bzw. des Videoprojektors 190 nicht ausreichend, um den Bio-Sensor-Chip 301/302 mit der gewünschten Auflösung (mindestens dem Abstand der Teststellen auf dem Chip) anzuregen, so kann der Chip zusätzlich abgescannt werden: Dazu wird das Bild des Beleuchtungssystems 100 nur auf einen Teil des Chips 301/302 verkleinert, so dass die gewünschte Auflösung erreicht wird. In einem ersten Schritt wird zunächst dieser Teil des Chips angeregt und ausgelesen. In einem Folgeschritt wird entweder der Chip über einen x-y-Verschiebetisch oder das Bild über bewegliche Umlenkspiegel um die Breite eines Bildes verschoben. Anschließend wird der dadurch neu ausgewählte Bereich des Chips angeregt und ausgelesen. Dieses Verfahren wird so lange wiederholt, bis der gesamte Chip 301/302 ausgelesen wurde.

## Patentansprüche

1. Verfahren zum Nachweis von organischen Molekülen, insbesondere von Biomolekülen und Polymeren, in einer Probensubstanz, mit folgenden Verfahrensschritten:
- Bereitstellen eines Untersuchungssystem (300) aus einem Array von Teststellen (T), wobei jede Teststelle spezifische Sondenmoleküle (320) aufweist;
- Bereitstellen eines Beleuchtungssystem (100) zur optischen Beleuchtung des Arrays von Teststellen, wobei das Beleuchtungssystem ein Array von getrennt ansteuerbaren Beleuchtungsquellen (B) umfasst, die so angeordnet sind, dass jeder Teststelle (T) eines Teilarrays des Untersuchungssystems (300) zumindest eine, im Wesentlichen nur sie beleuchtende Beleuchtungsquelle zugeordnet ist;
- Zuführen der Probensubstanz zu dem Untersuchungssystem (300);
- Beleuchten der Teststellen (T) durch Ansteuerung der ihnen jeweils zugeordneten Beleuchtungsquellen (B) nach einem vorgewählten Schema, bei dem in mindestens einem Schritt mehrere Teststellen gleichzeitig beleuchtet werden;
- Auslesen aller beleuchteten Teststellen gemeinsam nach jedem Schritt des Beleuchtungsschemas mit einem Detektionssystem (400) ohne räumliche Auflösung, und
- Identifizieren solcher Teststellen (T), deren Sondenmoleküle (320) mit den nachzuweisenden organischen Molekülen (350) wechselwirken.

2. Verfahren nach Anspruch 1, bei dem die Teststellen (T), deren Sondenmoleküle (320) mit den nachzuweisenden organischen Molekülen (350) wechselwirken, durch ein elektrochemisches Verfahren, insbesondere durch eine Strom-, Ladungs-, Spannungs-, Potentialmessung, cyclische Voltametrie, Amperometrie, oder eine Leitfähigkeitsmessung, identifiziert werden.

3. Verfahren nach Anspruch 1 oder 2, bei dem die Teststellen (T) auf zumindest einer leitfähigen Oberfläche angeordnet sind und die Teststellen, an denen eine Reaktion zwischen den Sondenmolekülen (320) und den nachzuweisenden organischen Molekülen (350) stattgefunden hat, durch eine Bestimmung der elektrischen Kommunikation zwischen den Teststellen und der leitfähigen Oberfläche identifiziert werden.

4. Verfahren nach Anspruch 1, bei dem die Teststellen (T), deren Sondenmoleküle (320) mit den nachzuweisenden organischen Molekülen (350) wechselwirken, durch ein optisches Verfahren, insbesondere durch Detektion von Lumineszenz, identifiziert werden.

5. Verfahren nach einem der Ansprüche 1 bis 4, bei dem das Schema zur Ansteuerung der Beleuchtungsquellen dadurch auf die Wahrscheinlichkeit der Wechselwirkung zwischen den Sondenmolekülen (320) der jeweiligen Teststelle (T) und den nachzuweisenden organischen Molekülen (350) abgestimmt wird, dass bei geringer Wechselwirkungswahrscheinlichkeit viele Teststellen (T) gleichzeitig beleuchtet werden und bei hoher Wechselwirkungswahrscheinlichkeit nur eine oder wenige Teststellen gleichzeitig beleuchtet werden.

6. Verfahren nach einem der Ansprüche 1 bis 5, bei dem das Schema zur Ansteuerung der Beleuchtungsquellen so gewählt wird, dass keine benachbarten Teststellen (T) gleichzeitig beleuchtet werden und/oder die Abstände zwischen gleichzeitig beleuchteten Teststellen möglichst groß sind.

7. Verfahren nach einem der Ansprüche 1 bis 6, bei dem die Teststellen (T) periodisch beleuchtet werden und die Identifizierung der Reaktion mit Hilfe frequenz- oder phasenspezifischer Signalverarbeitung erfolgt.

8. Verfahren nach einem der Ansprüche 1 bis 7, bei dem verschiedene Teststellen (T) periodisch mit unterschiedlichen Frequenzen oder Phasen beleuchtet werden und die Identifizierung der Reaktion mit Hilfe frequenz- oder phasenspezifischer Signalverarbeitung erfolgt.

9. Verfahren nach einem der Ansprüche 1 bis 8, bei dem die detektierten Signale mittels elektronischer Datenverarbeitung ausgewertet werden, insbesondere, wobei Korrekturen mittels für die Vorrichtung charakteristische Kenngrößen vornehmbar sind.

10. Verfahren nach einem der Ansprüche 1 bis 9, bei dem das Teilarray von Teststellen T das gesamte Teststellenarray umfasst.

11. Verfahren nach einem der Ansprüche 1 bis 9, bei dem das Teilarray von Teststellen T nur eine Teilmenge der Teststellen auf dem Untersuchungssystem (300) enthält und bei dem durch eine relative Bewegung zwischen Beleuchtungssystem (100) und Untersuchungssystem (300) sukzessive alle Teststellen des Untersuchungssystems beleuchtet und auf deren lichtinduzierte Reaktion überprüft werden.

12. Vorrichtung zur Durchführung des Verfahrens nach einem der Ansprüche 1 bis 11, mit
- einem Untersuchungssystem (300) aus einem Array von Teststellen (T), denen die Probensubstanz zuführbar ist, wobei jede Teststelle spezifische Sondenmoleküle (320) aufweist,
- einem Beleuchtungssystem (100) zur optischen Beleuchtung des Arrays von Teststellen (T), das ein Array von getrennt ansteuerbaren Beleuchtungsquellen (B) umfasst, die so angeordnet sind, dass jeder Teststelle (T) eines Teilarrays des Untersuchungssystems (300) zumindest eine, im Wesentlichen nur sie beleuchtende Beleuchtungsquelle zugeordnet ist,
- Mitteln zum Zuführen der Probensubstanz zu dem Untersuchungssystem (300),
- Mitteln zur Ansteuerung der den Teststellen (T) jeweils zugeordneten Beleuchtungsquellen (B) nach einem vorgewählten Schema, die ausgelegt und eingerichtet sind, in mindestens einem Schritt des Beleuchtungsschemas mehrere Teststellen (T) gleichzeitig zu beleuchten,
- einem Detektionssystem (400) ohne räumliche Auflösung zum Auslesen aller beleuchteten Teststellen (T) gemeinsam nach jedem Schritt des Beleuchtungsschemas, und
- Mitteln zum Identifizieren solcher Teststellen (T), deren Sondenmoleküle (320) mit den nachzuweisenden organischen Molekülen (350) wechselwirken.

13. Vorrichtung nach Anspruch 12, bei der das Detektionssystem (400) ein Strom-, Ladungs-, Spannungs-, Potentialmessgerät, ein Cyclovoltametrie-Gerät, ein Amperometrie-Gerät oder ein Leitfähigkeitsmessgerät umfasst.

14. Vorrichtung nach Anspruch 12 oder 13, bei der die Teststellen (T) auf zumindest einer leitfähigen Oberfläche angeordnet sind und das Detektionssystem (400) ein Messgerät (410) zur Bestimmung der elektrischen Kommunikation zwischen den Teststellen und der leitfähigen Oberfläche umfasst.

15. Vorrichtung nach Anspruch 12, bei der das Detektionssystem (400) einen optischen Detektor (450) zum optischen Nachweis einer lichtinduzierten Reaktion des Untersuchungssystems (300), insbesondere zum Nachweis von aus dem Untersuchungssystem emittierten Licht, umfasst.

16. Vorrichtung nach einem der Ansprüche 12 bis 15, bei der die Mittel zur Ansteuerung der Beleuchtungsquellen (B) nach einem vorgewählten Schema ausgelegt und eingerichtet sind, die Wahrscheinlichkeit der Wechselwirkung zwischen den Sondenmolekülen (320) der jeweiligen Teststelle (T) und den nachzuweisenden organischen Molekülen (350) dadurch zu berücksichtigen, dass sie bei geringer Wechselwirkungswahrscheinlichkeit viele Teststellen (T) gleichzeitig beleuchten und bei hoher Wechselwirkungswahrscheinlichkeit nur eine oder wenige Teststellen gleichzeitig beleuchten.

17. Vorrichtung nach einem der Ansprüche 12 bis 16, bei der die Mittel zur Ansteuerung der Beleuchtungsquellen (B) nach einem vorgewählten Schema so ausgelegt und eingerichtet sind, dass sie keine benachbarten Teststellen (T) gleichzeitig beleuchten und/oder in möglichst großem Abstand angeordnete Teststellen gleichzeitig zu beleuchten.

18. Vorrichtung nach einem der Ansprüche 12 bis 17, bei der die Mittel zur Ansteuerung der Beleuchtungsquellen (B) für eine periodische Beleuchtung der Teststellen (T) eingerichtet sind, und die Mittel zum Identifizieren der Teststellen (T), deren Sondenmoleküle (320) mit den nachzuweisenden organischen Molekülen (350) wechselwirken, frequenz- oder phasenspezifische Signalverarbeitungsmittel umfassen.

19. Vorrichtung nach einem der Ansprüche 12 bis 18, mit einem Datenverarbeitungssystem zum Speichern, Auswerten und Darstellen der nachgewiesenen Reaktionen

20. Vorrichtung nach einem der Ansprüche 12 bis 19, weiter umfassend ein optisches System (200) zum Abbilden des Beleuchtungssystems (100) auf das Untersuchungssystem (300), derart, dass auf jede Teststelle (T) des Teilarrays zumindest eine ihr zugeordnete Beleuchtungsquelle (B) abbildbar ist.

21. Vorrichtung nach einem der Ansprüche 12 bis 19, bei der das Beleuchtungssystem (100) im Wesentlichen parallel zu dem Untersuchungssystem (300) und in so geringem Abstand von diesem angeordnet ist, dass die von jeder einer Teststelle (T) zugeordneten Beleuchtungsquelle (B) emittierte Strahlung im Wesentlichen nur diese eine Teststelle beleuchtet.

22. Vorrichtung nach einem der Ansprüche 12 bis 21, bei der das Beleuchtungssystem (100) ein Beleuchtungselement (150,160) umfasst, ausgewählt aus der Gruppe bestehend aus einer Elektronenstrahlröhre, einem LCD-Anzeigenelement, einem TFT-LCD-Anzeigenelement, einem Feldemissions-Anzeigenelement, einem Elektrolumineszenz-Anzeigenelement, einem Plasmabildschirm, einem Polymer-Anzeigenelement, einem LED-Array, einem beleuchteten Flächenlichtmodulator (SLM), insbesondere einem mikromechanischen Spiegelarray (DMD), einer Vakuumfluoreszenzanzeige (VFD), einem Laserarray und einem Videoprojektor.

23. Vorrichtung nach einem der Ansprüche 12 bis 22, bei der das Teilarray von Teststellen T das gesamte Teststellenarray umfasst.

24. Vorrichtung nach einem der Ansprüche 12 bis 22, bei der das Teilarray von Teststellen (T) nur eine Teilmenge der Teststellen auf dem Untersuchungssystem (300) enthält und die zusätzlich Bewegungsmittel umfasst, die eine relative Bewegung zwischen Beleuchtungssystem und Untersuchungssystem ermöglichen, insbesondere einen Verschiebetisch oder einen beweglichen Umlenkspiegel.

## Claims

1. A method for detecting organic molecules, especially biomolecules and polymers, in a probe substance, with the following process steps:
- providing a test system (300) comprising an array of test sites (T), each test site having specific probe molecules (320);
- providing an illumination system (100) for optically illuminating the array of test sites, the illumination system comprising an array of independently addressable illumination sources (B) that are arranged such that to each test site (T) of a sub-array of the test system (300) is assigned at least one illumination source that illuminates substantially only it;
- supplying the probe substance to the test system (300);
- illuminating the test sites (T) by addressing the respective illumination sources (B) assigned to them, according to a preselected pattern wherein, in at least one step, multiple test sites are illuminated simultaneously;
- reading out all illuminated test sites together after each step of the illumination pattern with a detection system (400) having no spatial resolution; and
- identifying those test sites (T) whose probe molecules (320) interact with the organic molecules (350) to be detected.

2. The method according to claim 1, wherein the test sites (T) whose probe molecules (320) interact with the organic molecules (350) to be detected are identified by an electrochemical method, especially through a current, charge, voltage, or potential measurement, cyclic voltammetry, amperometry, or a conductivity measurement.

3. The method according to claim 1 or 2, wherein the test sites (T) are arranged on at least one conductive surface and the test sites at which a reaction between the probe molecules (320) and the organic molecules (350) to be detected occurred, are identified by determining the electrical communication between the test sites and the conductive surface.

4. The method according to claim 1, wherein the test sites (T) whose probe molecules (320) interact with the organic molecules (350) to be detected are identified by an optical method, especially through a detection of luminescence.

5. The method according to one of claims 1 through 4, wherein the pattem for addressing the illumination sources is selected depending on the probability of interaction between the probe molecules (320) of the respective test site (T) and the organic molecules (350) to be detected by simultaneously illuminating many test sites (T) if the probability of interaction is low, and simultaneously illuminating only one test site or a few test sites if the probability of interaction is high.

6. The method according to one of claims 1 through 5, wherein the pattern for addressing the illumination sources is selected such that no adjacent test sites (T) are illuminated simultaneously and/or the distances between simultaneously illuminated test sites are as large as possible.

7. The method according to one of claims 1 through 6, wherein the test sites (T) are illuminated periodically and the identification of the reaction is done utilizing frequency- or phase-specific signal processing.

8. The method according to one of claims 1 through 7, wherein various test sites (T) are illuminated periodically with differing frequencies or phases and the identification of the reaction is done utilizing frequency- or phase-specific signal processing.

9. The method according to one of claims 1 through 8, wherein the detected signals are evaluated by means of electronic data processing, in particular, such that corrections may be carried out using characteristic parameters of the apparatus.

10. The method according to one of claims 1 through 9, wherein the sub-array of test sites T comprises the entire test site array.

11. The method according to one of claims 1 through 9, wherein the sub-array of test sites (T) includes only a subset of the test sites on the test system (300) and wherein, through a relative movement between the illumination system (100) and the test system (300), all test sites of the test system are successively illuminated and examined for their light-induced reaction.

12. An apparatus for carrying out the method according to one of claims 1 through 11, comprising
- a test system (300) comprising an array of test sites (T) to which the probe substance can be supplied, each test site having specific probe molecules (320),
- an illumination system (100) for optically illuminating the array of test sites (T), comprising an array of independently addressable illumination sources (B) that are arranged such that to each test site (T) of a sub-array of the test system (300) is assigned at least one illumination source that illuminates substantially only it.
- means of supplying the probe substance to the test system (300),
- means of addressing, according to a preselected pattern, the respective illumination sources (B) assigned to the test sites (T), which means are arranged and adapted to simultaneously illuminate multiple test sites in at least one step of the illumination pattern,
- a detection system (400) having no spatial resolution, for reading out all illuminated test sites (T) together after each step of the illumination pattern, and
- means of identifying those test sites (T) whose probe molecules (320) interact with the organic molecules (350) to be detected.

13. The apparatus according to claim 12, wherein the detection system (400) comprises a current, charge, voltage, or potential measuring device, a cyclovoltammetry device, an amperometry device, or a conductivity measuring device.

14. The apparatus according to claim 12 or 13, wherein the test sites (T) are arranged on at least one conductive surface and the detection system (400) comprises a measuring device (410) for determining the electrical communication between the test sites and the conductive surface.

15. The apparatus according to claim 12, wherein the detection system (400) comprises an optical detector (450) for optically detecting a light-induced reaction of the test system (300), especially for detecting light emitted from the test system.

16. The apparatus according to one of claims 12 through 15, wherein the means for addressing the illumination sources (B) according to a preselected pattern is arranged and adapted to take into account the probability of interaction between the probe molecules (320) of the respective test site (T) and the organic molecules (350) to be detected by simultaneously illuminating many test sites (T) if the probability of interaction is low, and simultaneously illuminating only one test site or a few test sites if the probability of interaction is high.

17. The apparatus according to one of claims 12 through 16, wherein the means for addressing the illumination sources (B) according to a preselected pattern are arranged and adapted to not simultaneously illuminating adjacent test sites (T) and/or to simultaneously illuminating test sites arranged as far apart as possible.

18. The apparatus according to one of claims 12 through 17, wherein the means for addressing the illumination sources (B) are adapted to periodically illuminating the test sites (T), and the means for identifying the test sites whose probe molecules (320) interact with the organic molecules (350) to be detected comprise frequency- or phase-specific signal processing means.

19. The apparatus according to one of claims 12 through 18, having a data processing system for storing, evaluating, and displaying the detected reactions.

20. The apparatus according to one of claims 12 through 19, further comprising an optical system (200) for imaging the illumination system (100) on the test system (300) such that for each test site (T) of the sub-array, at least one of the illumination sources (B) assigned to it is imageable on said test site (T).

21. The apparatus according to one of claims 12 through 19, wherein the illumination system (100) is disposed substantially parallel to the test system (300) and at such a short distance therefrom that the radiation emitted from each illumination source (B) assigned to a test site (T) illuminates substantially only this one test site.

22. The apparatus according to one of claims 12 through 21, wherein the illumination system (100) comprises a illumination element (150,160) selected from the group consisting of a cathode ray tube, an LCD display element, a TFT-LCD display element, a field-emission display element, an electroluminescent display element, a plasma screen, a polymer display element, an LED array, an illuminated spatial light modulator (SLM), especially a digital micromirror device (DMD), a vacuum fluorescent display (VFD), a laser array, and a video projector.

23. The apparatus according to one of claims 12 through 22, wherein the sub-array of test sites T comprises the entire test site array.

24. The apparatus according to one of claims 12 through 22, wherein the sub-array of test sites (T) includes only a subset of the test sites on the test system (300) and, additionally, comprises movement means facilitating a relative movement between the illumination system and the test system, especially a translation table or a movable tilted mirror.

## Revendications

1. Procédé de détection de molécules organiques, en particulier de biomolécules et de polymères, dans une substance échantillon, avec les étapes de procédé suivantes :
- préparation d'un système d'analyse (300) constitué d'une matrice de lieux d'essai (T), chaque lieu d'essai présentant des molécules sondes (320) spécifiques ;
- préparation d'un système d'illumination (100) pour l'illumination optique de la matrice de lieux d'essai, le système d'illumination comprenant une matrice de sources d'illumination (B) pouvant être excitées de façon séparée, qui sont disposées de telle façon qu'à chaque lieu d'essai (T) d'une matrice partielle du système d'analyse (300) est associée au moins une source d'illumination qui n'illumine essentiellement que celui-ci ;
- introduction de la substance d'essai dans le système d'analyse (300) ;
- illumination des lieux d'essai (T) par excitation des sources d'illumination (B) qui leur sont respectivement associées, selon un schéma présélectionné, dans lequel plusieurs lieux d'essai sont illuminés simultanément en au moins une étape ;
- lecture de tous les lieux d'essai illuminés conjointement après chaque étape du schéma d'illumination, avec un système de détection (400) sans résolution spatiale, et
- identification des lieux d'essai (T) dont les molécules sondes (320) interagissent avec les molécules organiques (350) à détecter.

2. Procédé selon la revendication 1, dans lequel les lieux d'essai (T) dont les molécules sondes (320) interagissent avec les molécules organiques (350) à détecter, sont identifiés par un procédé électrochimique, en particulier par une mesure de courant, de charge, de tension, de potentiel, une voltamétrie cyclique, une ampérométrie, ou une mesure de conductivité.

3. Procédé selon la revendication 1 ou 2, dans lequel les lieux d'essai (T) sont disposés sur au moins une surface conductrice et les lieux d'essai, sur lesquels a eu lieu une réaction entre les molécules sonde (320) et les molécules organiques (350) à détecter, sont identifiés par une détermination de la communication électrique entre les lieux d'essai et la surface conductrice.

4. Procédé selon la revendication 1, dans lequel les lieux d'essai (T), dont les molécules sondes (320) interagissent avec les molécules organiques (350) à détecter, sont identifiés par un procédé optique, en particulier par détection de luminescence.

5. Procédé selon l'une quelconque des revendications 1 à 4, dans lequel le schéma pour l'excitation des sources d'illumination est ajusté sur la probabilité de l'interaction entre les molécules sondes (320) du lieu d'essai (T) en question et les molécules organiques (350) à détecter, en illuminant simultanément un grand nombre de lieux d'essai (T) dans le cas d'une faible probabilité d'interaction, et en illuminant simultanément seulement un ou quelques lieux d'essai dans le cas d'une probabilité élevée d'interaction.

6. Procédé selon l'une quelconque des revendications 1 à 5, dans lequel le schéma pour exciter les sources d'illumination est choisi de telle sorte que des lieux d'essai voisins (T) ne soient pas illuminés simultanément, et/ou que les distances entre les lieux d'essai illuminés simultanément soient si possible grandes.

7. Procédé selon l'une quelconque des revendications 1 à 6, dans lequel les lieux d'essai (T) sont illuminés périodiquement et l'identification de la réaction se réalise à l'aide d'un traitement de signal spécifique à la fréquence ou à la phase.

8. Procédé selon l'une quelconque des revendications 1 à 7, dans lequel différents lieux d'essai (T) sont illuminés périodiquement avec des fréquences ou des phases différentes, et l'identification de la réaction se réalise à l'aide d'un traitement de signal spécifique à la fréquence ou à la phase.

9. Procédé selon l'une quelconque des revendications 1 à 8, dans lequel les signaux détectés sont évalués au moyen d'un traitement de données électronique, en particulier, des corrections pouvant être effectuées au moyen de grandeurs caractéristiques pour le dispositif.

10. Procédé selon l'une quelconque des revendications 1 à 9, dans lequel la matrice partielle de lieux d'essai T comprend la matrice entière des lieux d'essai.

11. Procédé selon l'une quelconque des revendications 1 à 9, dans lequel la matrice partielle des lieux d'essai T ne contient qu'un ensemble partiel des lieux d'essai sur le système d'analyse (300) et dans lequel tous les lieux d'essai du système d'analyse sont illuminés successivement à l'aide d'un mouvement relatif entre le système d'illumination (100) et le système d'analyse (300), et contrôlés quant à leur réaction induite par la lumière.

12. Dispositif pour réaliser le procédé selon l'une quelconque des revendications 1 à 11, avec
- un système d'analyse (300) constitué d'une matrice de lieux d'essai (T) dans laquelle la substance échantillon peut être introduite, chaque lieu d'essai présentant des molécules sonde (320) spécifiques,
- un système d'illumination (100) pour l'illumination optique de la matrice de lieux d'essai (T), qui comprend une matrice de sources d'illumination (B) pouvant être excitées de façon séparée, lesquelles étant disposées de telle façon qu'à chaque lieu d'essai (T) d'une matrice partielle du système d'analyse (300) est associé au moins une source d'illumination qui n'illumine essentiellement que celui-ci,
- des moyens pour introduire la substance échantillon dans le système d'analyse (300),
- des moyens pour exciter les sources d'illumination (B) associées respectivement aux lieux d'essai (T) selon un schéma présélectionné, lesquelles sont ajustées et configurées, afin d'illuminer simultanément plusieurs lieux d'essai (T) en au moins une étape,
- un système de détection (400) sans résolution spatiale pour lire conjointement tous les lieux d'essai (T) illuminés, après chaque étape du schéma d'illumination, et
- des moyens pour identifier les lieux d'essai (T), dont les molécules sondes (320) interagissent avec les molécules organiques (350) à détecter.

13. Dispositif selon la revendication 12, dans lequel le système de détection (400) comprend un appareil de mesure de courant, de charge, de tension, de potentiel, un appareil de voltamétrie cyclique, un appareil d'ampérométrie ou un appareil de mesure de conductivité.

14. Dispositif selon la revendication 12 ou 13, dans lequel les lieux d'essai (T) sont disposés sur au moins une surface conductrice, et le système de détection (400) comprend un appareil de mesure (410) pour déterminer la communication électrique entre les lieux d'essai et la surface conductrice.

15. Dispositif selon la revendication 12, dans lequel le système de détection (400) comprend un détecteur optique (450) pour la détection optique d'une réaction induite par la lumière, du système d'analyse (300), en particulier pour la détection de la lumière émise à partir du système d'analyse.

16. Dispositif selon l'une quelconque des revendications 12 à 15, dans lequel les moyens pour exciter les sources d'illumination (B) sont ajustés et configurés de façon à prendre en compte la probabilité de l'interaction entre les molécules sondes (320) du lieu d'essai respectif (T) et les molécules organiques (350) à détecter, en illuminant simultanément de nombreux lieux d'essai (T) dans le cas d'une faible probabilité d'interaction et en illuminant simultanément seulement un ou quelques lieux d'essai dans le cas d'une probabilité élevée d'interaction.

17. Dispositif selon l'une quelconque des revendications 12 à 16, dans lequel les moyens pour exciter les sources d'illumination (B) selon un schéma présélectionné, sont ajustés et configurés de sorte à ne pas illuminer simultanément des lieux d'essai (T) voisins, et/ou à illuminer simultanément des lieux d'essai disposés si possible à grande distance l'un de l'autre.

18. Dispositif selon l'une quelconque des revendications 12 à 17, dans lequel les moyens pour exciter les sources d'illumination (B) sont ajustés pour illuminer périodiquement des lieux d'essai (T), et les moyens pour identifier les lieux d'essai (T) dont les molécules sondes (320) interagissent avec les molécules organiques (350) à détecter, comprennent des moyens de traitement de signal spécifiques à la fréquence ou à la phase.

19. Dispositif selon l'une quelconque des revendications 12 à 18, avec un système de traitement de données pour mémoriser, évaluer et représenter les réactions détectées.

20. Dispositif selon l'une quelconque des revendications 12 à 19, comprenant en outre un système optique (200) pour reproduire le système d'illumination (100) sur le système d'analyse (300), de sorte à pouvoir reproduire sur chaque lieu d'essai (T) de la matrice partielle, au moins une source d'illumination qui lui est associée (B).

21. Dispositif selon l'une quelconque des revendications 12 à 19, dans lequel le système d'illumination (100) est essentiellement disposé parallèlement an système d'analyse (300) et à une distance de celui-ci tellement faible que le rayonnement émis à partir de chaque source d'illumination (B) associée à un lieu d'essai (T) n'illumine essentiellement que ledit un lieu d'essai.

22. Dispositif selon l'une quelconque des revendications 12 à 21, dans lequel le système d'illumination (100) comprend un élément d'illumination (150, 160), choisi dans le groupe formé par un tube à faisceau électronique, un élément d'affichage à cristaux liquides (LCD), un élément d'affichage LCD à couche mince de transistors (TFT), un élément d'affichage à émission de champ, un élément d'affichage à électroluminescenee, un écran à plasma, un élément d'affichage à polymère, une matrice de diodes électroluminescentes, un modulateur spatial de lumière (SLM) illuminé, en particulier une matrice de miroirs micromécaniques (DMD), un affichage fluorescent à vide (VFD), une matrice de lasers, et un projecteur vidéo.

23. Dispositif selon l'une quelconque des revendications 12 à 22, dans lequel la matrice partielle de lieux d'essai T comprend la matrice entière de lieux d'essai.

24. Dispositif selon l'une quelconque des revendications 12 à 22, dans lequel la matrice partielle de lieux d'essai (T) ne renferme qu'un ensemble partiel des lieux d'essai sur le système d'analyse (300) et qui comprend en plus des moyens de déplacement qui permettent un déplacement relatif entre le système d'illumination et le système d'analyse, en particulier une table de déplacement ou un miroir de déviation mobile.
